# EUROPEAN PATENT SPECIFICATION

(11) **EP 2 398 026 B1**
(45) Date of publication and mention of the grant of the patent: **21.08.2019**
(21) Application number: 11170034.0
(22) Date of filing: 15.06.2011
(51) Int. Cl.: H01G 4/35, A61N 1/375, H01G 4/232

(54) **COATING OF NON-SOLDERABLE BASE METAL FOR SOLDERING APPLICATION IN MEDICAL DEVICE COMPONENT**
BESCHICHTUNG VON NICHT LÖTBAREM GRUNDMETALL FÜR EINE LÖTANWENDUNG IN MEDIZINISCHER VORRICHTUNGSKOMPONENTE
REVÊTEMENT DE MÉTAL À BASE NE POUVANT PAS ÊTRE SOUDÉ POUR UNE APPLICATION À SOUDER DANS UN COMPOSANT DE DISPOSITIF MÉDICAL

(30) Priority: 15.06.2010 US 354747 P
(43) Date of publication of application: 21.12.2011
(73) Proprietor: Greatbatch Ltd., Clarence, New York 14031 (US)
(72) Inventor: Sutay, Todd C., Warsaw, NY 14569 (US); Seitz, Keith, Clarence Center, NY 14032 (US); Hussein, Haytham, Orchard Park, NY 14127 (US); Thanawala, Sachin, Lancaster, NY 14086 (US); Marzano, Thomas, East Amherst, NY 14051 (US)
(74) Representative: SSM Sandmair

(56) References cited:
- EP-A2- 1 796 115
- US-A- 5 406 444
- US-A- 6 159 560
- US-A1- 2005 060 003

## Description

### CROSS-REFERENCE TO RELATED APPLICATIONS

This application claims priority from U.S. Provisional Patent Application Serial No. 61/354,747 filed June 15, 2010.

### BACKGROUND OF THE INVENTION

### 1. Field of the Invention

This invention relates generally to a hermetic feedthrough terminal pin assembly, preferably of the type incorporating a filter capacitor. More specifically, this invention relates to terminal pins comprising a refractive metal core in which an electrically conductive second metal is selectively coated to provide a cost effective terminal pin of increased solderability for incorporation into a feedthrough filter capacitor assembly, particularly of the type used in implantable medical devices such as cardiac pacemakers, cardioverter defibrillators, and the like, to decouple and shield internal electronic components of the medical device from undesirable electromagnetic interference (EMI) signals. The terminal pin feedthrough assembly provides a hermetic seal that prevents passage or leakage of fluids into the medical device.

### 2. Prior Art

Feedthrough assemblies are generally well known in the art for use in connecting electrical signals through the housing or case of an electronic instrument. For example, in an implantable medical device, such as a cardiac pacemaker, defibrillator, or neurostimulator, the feedthrough assembly comprises one or more conductive terminal pins supported by an insulator structure for passage of electrical signals from the exterior to the interior of the medical device. The conductive terminals are fixed into place using a gold brazing process, which provides a hermetic seal between the pin and insulative material. Conventionally, the terminal pins have been composed of platinum or a combination of platinum and iridium. Platinum and platinum-iridium alloys are biocompatible, have good mechanical strength, which adds to the durability of the feedthrough. However, platinum is a precious metal that creates a manufacturing cost barrier.

The replacement of platinum and platinum alloys by refractive metals such as niobium, molybdenum and tungsten offers several advantages. First, these refractive metals have a significant cost advantage over platinum. Secondly, these refractive metals are generally known to be biocompatible. Finally, previous research has shown that after high temperature brazing, there is no significant degradation in the mechanical properties of these refractive metals, in comparison to platinum.

However, these refractive metals are susceptible to surface oxidation. Surface oxidation generally inhibits the ability of these metals to be joined to other materials, particularly other electrically conductive metals. What is needed, therefore, is a biocompatible, mechanically robust, cost effective terminal pin that can be readily joined to other metals. The present invention provides embodiments by which a terminal pin of a cost effective core metal is selectively coated with a metal that can be more readily joined to other electrically conductive metals.

EP 1 796 115 A2 discloses feedthrough filter capacitor assemblies having low cost terminal pins. The terminal pins comprise an outer coating of palladium coating a core material other than of palladium. The feedthrough filter capacitor assemblies are particularly useful for incorporation into implantable medical devices such as cardiac pacemakers, cardioverter defibrillators, and the like, to decouple and shield internal electronic components of the medical device from undesirable electromagnetic interference (EMI) signals.

US 5,406,444 A discloses a feedthrough assembly in which a thin film protective conductive metal coating may be deposited over the entire tantalum pin or may be deposited only to that portion of the terminal pin which is to be contacted by the capacitor.

US 2005/0060003 A1 relates to an electrical feedthrough comprising a terminal pin core of a first electrically conductive material and an external outer coating of a second electrically conductive metal supported on discrete portions of the terminal pin core.

### SUMMARY OF THE INVENTION

The invention is defined by a feedthrough assembly according to claim 1 and a method for providing a feedthrough assembly according to claim 15.

In a preferred form, a feedthrough filter capacitor assembly according to the present invention comprises an outer ferrule hermetically sealed to either an alumina insulator or fused glass dielectric material seated within the ferrule. The insulative material is also hermetically sealed to at least one terminal pin. That way, the feedthrough assembly prevents leakage of fluid, such as body fluid in a human implant application, past the hermetic seal at the insulator/ferrule and insulator/terminal pin interfaces.

According to an embodiment of the invention, the terminal pin of a feedthrough assembly, and preferably of a feedthrough filter capacitor assembly, is composed of a refractive metal core in which a layer of a non-refractive electrically conductive second metal is selectively contacted to the surface of the pin core. In a preferred embodiment, the terminal pin comprises a core of tantalum, niobium, molybdenum or alloy thereof. A layer of a second non-refractive electrically conductive metal, such as palladium, platinum, gold or silver, is selectively applied to a portion or portions of the surface of the core metal. In that respect, the application of the second electrically conductive metal is an alternative solderable, oxidation resistant material that provides a considerably less expensive terminal pin than conventional platinum or platinum-iridium terminal pins while still achieving the same benefits of biocompatibility, good mechanical strength, solderability and a reliable hermetic feedthrough seal.

These and other objects and advantages of the present invention will become increasingly more apparent by a reading of the following description in conjunction with the appended drawings.

### BRIEF DESCRIPTION OF THE DRAWINGS

FIG. 1 is a perspective view of a feedthrough assembly embodying the novel features of the present invention.
FIG. 2 is cross-sectional view of the feedthrough assembly of the present invention taken along line 2-2 of FIG. 1.
FIG. 3 is a side view of a preferred embodiment of a selectively coated terminal pin.
FIG. 4 is a cross-sectional view of a coated portion of the terminal pin taken along line 4-4 of FIG. 3.
FIG. 5 is a cross-sectional view of the feedthrough assembly of the present invention taken along line 5-5 of FIG. 2.
FIG. 6 is a cross-sectional view of the feedthrough assembly taken along line 6-6 of FIG. 2.

### DETAILED DESCRIPTION OF THE PREFERRED EMBODIMENTS

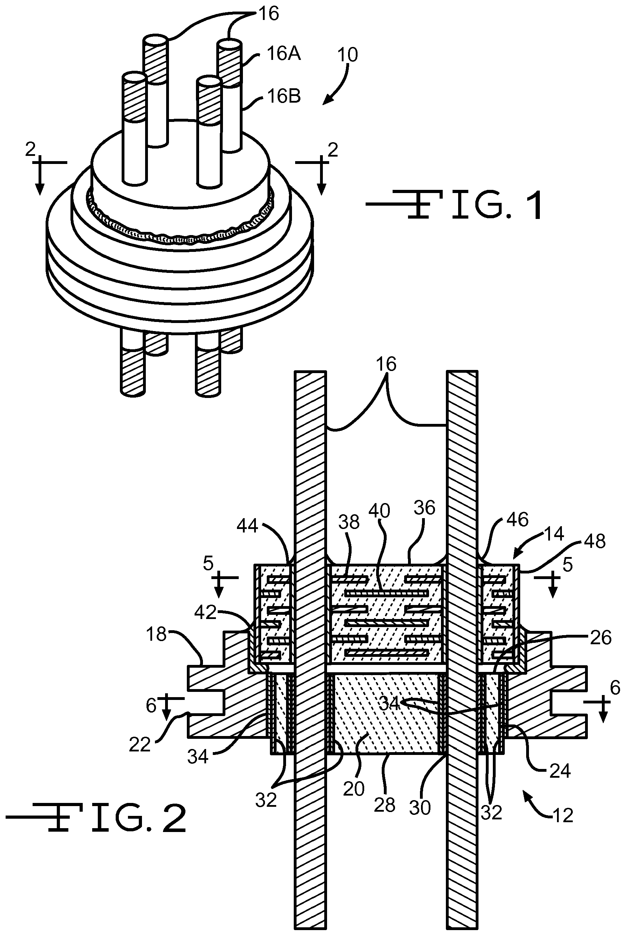

Referring now to the drawings, FIGS. 1 and 2 show an internally grounded feedthrough capacitor assembly 10 comprising a feedthrough 12 supporting a filter discoidal capacitor 14. The feedthrough filter assembly 10 is useful with medical devices, preferably implantable devices such as pacemakers, cardiac defibrillators, cardioverter defibrillators, cochlear implants, neurostimulators, internal drug pumps, deep brain stimulators, hearing assist devices, incontinence devices, obesity treatment devices, Parkinson's disease therapy devices, bone growth stimulators, and the like. The feedthrough 12 portion of the assembly 10 includes terminal pins 16 that provide for coupling, transmitting and receiving electrical signals to and from a patient's heart, while hermetically sealing the interior of the medical instrument against ingress of patient body fluids that could otherwise disrupt instrument operation or cause instrument malfunction. While not necessary for accomplishing these functions, it is desirable to attach the filter capacitor 14 to the feedthrough 12 for suppressing or decoupling undesirable EMI signals and noise transmission into the interior of the medical device.

More particularly, the feedthrough 12 of the feedthrough filter capacitor assembly 10 comprises a ferrule 18 defining an insulator-receiving bore surrounding an insulator 20. Suitable electrically conductive materials for the ferrule 18 include titanium, tantalum, niobium, stainless steel or combinations of alloys thereof, the former being preferred. The ferrule 18 may be of any geometry, non-limiting examples being round, rectangle, and oblong. A surrounding flange 22 extends from the ferrule 18 to facilitate attachment of the feedthrough 12 to the casing (not shown) of, for example, one of the previously described implantable medical devices. The method of attachment may be by laser welding or other suitable methods.

The insulator 20 is of a ceramic material such as of alumina, zirconia, zirconia toughened alumina, aluminum nitride, boron nitride, silicon carbide, glass or combinations thereof. Preferably, the insulating material is alumina, which is highly purified aluminum oxide, and comprises a sidewall 24 extending to a first upper side 26 and a second lower side 28. The insulator 20 is also provided with bores 30 that receive the terminal pins 16 passing there through. A layer of metal 32, referred to as metallization, is applied to the insulator sidewall 24 and the sidewall of the terminal pin bores 30 to aid a braze material 34 in hermetically sealing between the ferrule 18 and the insulator 20 and between the terminal pins 16 and the insulator 20, respectively.

Suitable metallization materials 32 include titanium, titanium nitride, titanium carbide, iridium, iridium oxide, niobium, tantalum, tantalum oxide, ruthenium, ruthenium oxide, zirconium, gold, palladium, molybdenum, silver, platinum, copper, carbon, carbon nitride, and combinations thereof. The metallization layer may be applied by various means including, but not limited to, sputtering, electron-beam deposition, pulsed laser deposition, plating, electroless plating, chemical vapor deposition, vacuum evaporation, thick film application methods, and aerosol spray deposition, and thin cladding.

Non-limiting examples of braze materials include gold, gold alloys, and silver. Then, if the feedthrough 12 is used where it will contact bodily fluids, the resulting brazes do not need to be covered with a biocompatible coating material. In other embodiments, if the brazes are not biocompatible, for example, if they contain copper, they are coated with a layer/coating of biocompatible/biostable material. Broadly, the biocompatibility requirement is met if contact of the braze/coating with body tissue and blood results in little or no immune response from the body, especially thrombogenicity (clotting) and encapsulation of the electrode with fibrotic tissue. The biostability requirement means that the braze/coating remains physically, electrically, and chemically constant and unchanged over the life of the patient.

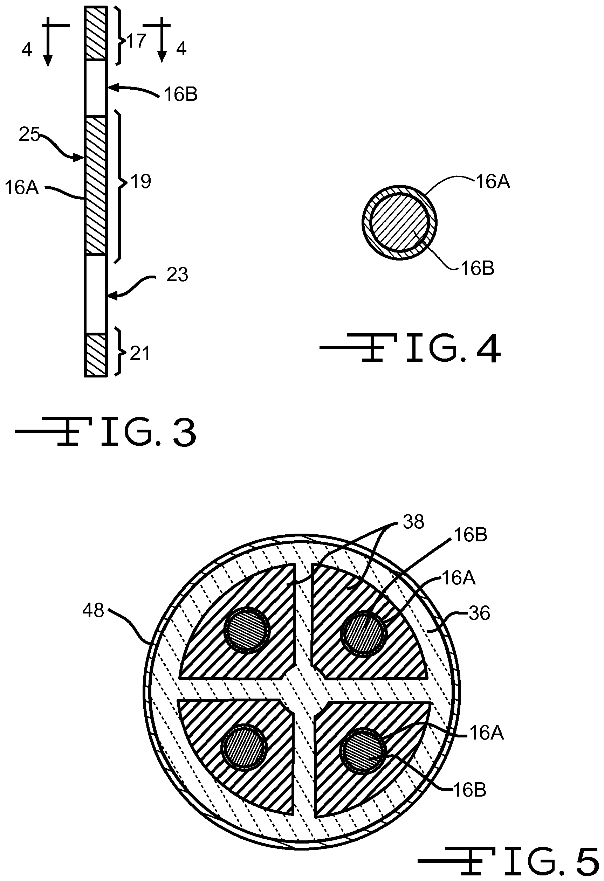

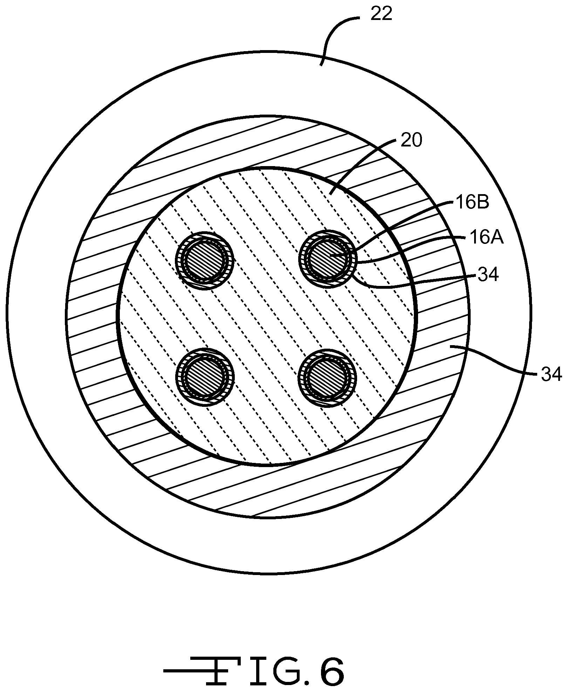

In the present invention, the terminal pins 16 (FIGS. 3, 4, 5 and 6) comprise a terminal pin core 16B of a first electrically conductive material and an exterior outer coating 16A of a second electrically conductive material. In a more preferred embodiment of the invention, the terminal pins 16 comprise a core 16B of a refractive metal and an exterior outer coating 16A comprising palladium and its alloys. Non-limiting examples include pure palladium and alloys comprising from about 50% to about 99% palladium along with other elements including those from the platinum group such as ruthenium, rhenium, and iridium, or refractory metals such as molybdenum, and boron, and combinations thereof.

Mechanical properties of the terminal pin 16 can be tailored to a desired mechanical performance by adjusting the amounts of the elemental additions in the palladium alloy. For example, age hardening can be improved by increasing the amount of ruthenium. Other additions to the palladium alloy such as platinum, gold, copper, and zinc, for example, increase the alloy's ability to achieve a higher tensile strength.

As previously mentioned, the terminal pin core 16B is comprised of a refractive metal. A refractory metal is herein defined as a metal that is resistant to heating and has a melting temperature greater than about 1,800°C. Non-limiting examples of refractory metals include niobium, molybdenum, tantalum, tungsten, rhenium, titanium, vanadium, zirconium, hafnium, osmium, iridium, and alloys thereof. In a more preferred embodiment, the terminal pin core 16B comprises niobium and niobium alloys. However, in an alternative embodiment, the terminal pin core 16B may comprise nickel-titanium (NITINOL®), titanium, particularly beta titanium, titanium alloys, stainless steel, palladium and palladium alloys, and combinations thereof.

In a preferred embodiment, the external outer coating 16A comprises an alternative electrically conductive metal. Non-limiting examples of this alternative second conductive metal comprise platinum, gold, silver, nickel and combinations thereof.

In a preferred embodiment, this second electrically conductive metal may have a surface 25 that is readily joinable to other materials, particularly electrically conductive metals. These material-joining processes may include soldering, welding and/or brazing. Preferably, the surface 25 of the second metal is "wettable" to tin based solders, such as Sn63/Pb37 and the like. A "wettable" surface is herein defined as the ability of a material to adhere to the surface.

As shown in FIGS. 1, 3 and 4, the external outer coating 16A of the second electrically conductive metal is selectively applied at discrete locations to a surface 23 of the terminal pin core 16B. The external outer coating 16A of the second electrically conductive metal is applied to discrete portions of the surface 23 of the terminal pin core 16B. These portions may include but are not limited to a distal end portion 21, a central portion 19 and/or a proximal end portion 17 of the terminal pin core 16B.

The means of coating may include sputtering, cladding, and or plating. The coating may be applied through a process of sputtering, electron-beam deposition, pulsed laser deposition, plating, electroless plating, chemical vapor deposition, vacuum evaporation, thick film application methods, aerosol spray deposition, and thin cladding.

Such a selectively applying the exterior outer coating 16A enhances electrical conduction and retards oxidation of the surface 23 of the terminal pin core 16B within these regions 17, 19, 21. Selectively applying the external outer coating 16A to the core 16B allows for improved design and manufacturing flexibility. For example, the coating 16A may be precisely applied to the surface 23 of the core 16B after high temperature processing. This feature is beneficial the external outer coating 16A can be tailored to meet the dimensions of the joining metal. Furthermore, the application of the external outer coating 16A to a discrete portion of the core 16B further reduces cost of manufacture.

As illustrated in FIGS. 1, 3, the portions 17,19,21 of the external outer coating 16A are not limited to a single external outer coating 16A composition. For example, the proximal end portion 17 of the terminal pin core 16B may be coated with a metal that is of a composition that is different then the coating 16A comprising the distal end portion 21 and/or the central portion 19. This feature of designing an external outer coating 16A of multiple compositions allows for custom tailoring of electrical or joining properties. The proximal and distal end portions 17, 21, each have a length of about 5 percent to about 15 percent of the total length of the terminal pin 16 may be coated with an outer external coating 16A of composition "A" which is preferable for soldering. The central portion 19 of the terminal pin core 16B, located within the capacitor 14, and having a length of from about 10 percent to about 40 percent of the total length of the terminal pin 16, may be coated with composition "B" which is readily joined to the metallization material by soldering, and the like, to provide improved electrical conduction or EMI filtration performance.

For example, it is known that refractive metals such as niobium, tungsten and molybdenum readily oxidize. This means that when it is used as a terminal pin material, secondary operations are necessary in order to effect a hermetic braze with low equivalent series resistance (ESR) characteristics. Providing a palladium outer coating 16A over a niobium core 16B in an evacuated atmosphere prior to formation of niobium oxide ensures that the thusly constructed terminal pin can be directly brazed into the insulator 20.

Although the terminal pin 16 is shown having a circular cross-section, that is not necessary. The terminal pin 16 can have other cross-sectional shapes including square, triangular, rectangular, and hexagonal, among others. Nonetheless, the core 16B has a diameter of from about **5,08** · **10⁻⁵ m** (0.002 inches) to about **5,08** · **10⁻⁴ m** (0.020 inches) and the outer coating 16A has a thickness of from about **1,27** · **10⁻⁸ m** (0.5 µ inches) to about **5,08** · **10⁻⁵ m** (0.002 inches).

Up to now, terminal pins for feedthrough assemblies used in implantable medical devices, and the like, have generally consisted of platinum. However, replacement of platinum and platinum alloys by such alternative metals as palladium and its alloys offers several advantages. For one, the density of platinum is 21.45 g/cc in comparison to palladium at 12.02 g/cc. Both of these materials are priced by weight, but used by volume. Therefore, palladium has significant cost advantage over platinum. Secondly, palladium has comparable electrical conductivity to platinum (platinum = 94.34 1/mohm-cm, palladium = 94.8 1/mohm-cm and gold = 446.4 1/mohm-cm). Thirdly, palladium and platinum have significantly equivalent mechanical properties. After high temperature brazing, there is no significant degradation of mechanical properties such as strength and elongation. Fourthly, palladium is both solderable and weldable. Fifthly, palladium has good radiopacity characteristics. This is an important consideration for viewing the terminal pin during diagnostic scans such as fluoroscopy. Lastly, but every bit as important, palladium is biocompatible. Previous research indicates a variety of positive biocompatibility studies (both soft tissue and bone) for all elements used. Palladium and its alloy additives are regarded as chemically inactive.

As further shown in FIGS. 1 and 3, the feedthrough filter capacitor 10 includes the filter capacitor 14 that provides for filtering undesirable EMI signals before they can enter the device housing via the terminal pins 16. The filter capacitor 14 comprises a ceramic or ceramic-based dielectric monolith 36 having multiple capacitor-forming conductive electrode plates formed therein. The capacitor dielectric 36 preferably has a circular cross-section matching the cross-section of the ferrule 18 and supports a plurality of spaced-apart layers of first or "active" electrode plates 38 in spaced relationship with a plurality of spaced apart layers of second or "ground" electrode plates 40. The filter capacitor 14 is preferably joined to the feedthrough 12 adjacent to the insulator side 26 by an annular bead 42 of conductive material, such as a solder or braze ring, or a thermal-setting conductive adhesive, and the like. The dielectric 36 includes lead bores 44 provided with an inner surface metallization layer. The terminal pins 16 pass there through and are conductively coupled to the active plates 38 by a conductive braze material 46 contacting between the terminal pins 16 and the bore metallization. In a similar manner, the ground plates 40 are electrically connected through an outer surface metallization 48 and the conductive material 42 to the ferrule 18.

It is appreciated that various modifications to embodiments described herein may be apparent to those of ordinary skill in the art without departing from the scope of the present invention as defined by the appended claims.

## Claims

1. A feedthrough assembly (10), which comprises:
a) an insulator (20) of electrically non-conductive material having a height defined by an insulator sidewall (24) extending to a first insulator end (26) and a second insulator end (28), wherein the insulator has at least one terminal pin bore (30) extending from the first end to the second end (28) thereof;
b) a terminal pin (16) received in the terminal pin bore (30), the terminal pin (16) having a sidewall extending to opposed first and second ends disposed spaced from the respective first and second insulator ends (26, 28); and
c) a ferrule (18) of an electrically conductive material and comprising a ferrule opening defined by a surrounding sidewall extending to a first ferrule end and a second ferrule end, wherein the insulator (20) is supported in the ferrule opening, **characterized by**
d) the terminal pin (16) comprising a terminal pin core (16B) of a first electrically conductive material and an external outer coating (16A) of a second electrically conductive material selectively applied to and directly supported on discrete portions of the terminal pin core (16B), wherein one discrete portion lies within a terminal pin proximal end portion (17), positioned between the first insulator end (26) and the first terminal pin end, or within a terminal pin distal end portion (21), positioned between the second insulator end (28) and the second end of the terminal pin (16), wherein the external outer coating (16A) comprises multiple compositions for multiple discrete portions, respectively; and
e) a first braze material contacting the coating of the second electrically conductive material on the terminal pin core thereby hermetically sealing the terminal pin to the insulator and a second braze material hermetically sealing the insulator to the ferrule.

2. The feedthrough assembly (10) of claim 1, wherein the first electrically conductive material of the terminal pin core (16B) is selected from the group consisting of niobium, tantalum, nickel-titanium, titanium, particularly beta titanium, titanium alloys, stainless steel, molybdenum, tungsten, platinum, and combinations thereof.

3. The feedthrough assembly (10) of claims 1 or 2, wherein the first electrically conductive material is left uncovered by the external outer coating (16A) where the second electrically material is not present.

4. The feedthrough assembly (10) of claims 1-3, wherein the second electrically conductive material contacts from 5 percent to 15 percent of a total length of the terminal pin (16) where it resides, at least one portion residing at either the proximal or distal end of the terminal pin.

5. The feedthrough assembly (10) of claims 1-3, wherein the second electrically conductive material contacts from 10 percent to 40 percent of a total length of the terminal pin (16) where it is hermetically sealed to the insulator (20).

6. The feedthrough assembly (10) of claims 1-3, wherein the second electrically conductive material contacting the proximal portion (17) is of a different composition than that of the second electrically conductive material contacting the distal (21) or central portion (19) of the terminal pin.

7. The feedthrough assembly (10) of claims 1-3 or 5, wherein the terminal pin core (16B) has a diameter of from 5.08 X 10⁻⁵ m to 5.08 X 10⁻⁴ m (0.002 inches to 0.020 inches).

8. The feedthrough assembly (10) of claims 1-3, 5 or 7, wherein the second electrically conductive material is a metal selected from the group consisting of palladium, palladium alloys, platinum, gold, silver, nickel and combinations thereof.

9. The feedthrough assembly (10) of claims 1-3, 5 or 7-8, wherein the external outer coating (16A) of the second electrically conductive material of the terminal pin (16) has a thickness of from 1.27 X 10⁻⁸ m to 7.62 X 10⁻⁵ m (0.5µ inches to 0.003 inches).

10. The feedthrough assembly (10) of claims 1-3, 5 or 7-9, wherein the terminal pin (16) has a cross-sectional shape selected from the group consisting of circular, square, rectangular, and hexagonal.

11. The feedthrough assembly (10) of claims 1-3, 5 or 7-10, wherein the insulator (20) is selected from the group consisting of alumina, zirconia, zirconia toughened alumina, aluminum nitride, boron nitride, silicon carbide, glass, and mixtures thereof.

12. The feedthrough assembly (10) of claims 1-3, 5 or 7-11, wherein the electrically conductive material of the ferrule (18) is selected from the group consisting of titanium, tantalum, niobium, stainless steel, and combinations of alloys thereof.

13. The feedthrough assembly (10) of claims 1-3, 5 or 7-12, wherein the first and second braze materials (34) are selected from the group consisting of gold, gold alloys, and silver.

14. The feedthrough assembly (10) of claims 1-3, 5 or 7-13 further including a metallization material (32) covering the insulator sidewall (24) and the terminal pin bore (30), the metallization material (32) selected from the group consisting of titanium, titanium nitride, titanium carbide, iridium, iridium oxide, niobium, tantalum, tantalum oxide, ruthenium, ruthenium oxide, zirconium, gold, palladium, molybdenum, silver, platinum, copper, carbon, carbon nitride, and mixtures thereof.

15. A method for providing a feedthrough assembly (10) according to any of claims 1-14 comprising a terminal pin (16), comprising the steps of:
a) providing a terminal pin core (16B) of a first electrically conductive material; and
b) coating a discrete portion of the terminal pin core (16B) with an external outer coating (16A) of a second electrically conductive material to a thickness such that the second electrically conductive material is not completely diffused into the first electrically conductive material of the terminal pin core (16B), wherein one discrete portion lies within a terminal pin proximal end portion (17), positioned between the first insulator end (26) and the first end of the terminal pin (16), or within a terminal pin distal end portion (21), positioned between the second insulator end (28) and the second end of the terminal pin (16).

16. The method of claim 15, including coating the second electrically conductive material over the terminal pin core (16B) using a process selected from the group consisting of sputtering, electron-beam deposition, pulsed laser deposition, plating, electroless plating, chemical vapor deposition, vacuum evaporation, thick film application methods, aerosol spray deposition, and thin cladding.

17. The method of claims 15 or 16, including selecting the first electrically conductive material of the terminal pin core (16B) from the group consisting of niobium, tantalum, nickel titanium, titanium, particularly beta titanium, titanium alloys, stainless steel, molybdenum, tungsten, platinum, and combinations thereof.

18. The method of claims 15-17, including providing the terminal pin core (16B) having a diameter of from 5.08 X 10⁻⁵ m to 5.08 X 10⁻⁴ m (0.002 inches to 0.020 inches).

19. The method of claims 15-18, including providing the outer coating (16A) of the second electrically conductive material being selected from the group consisting of palladium, palladium alloys, gold, silver, nickel, platinum, and combinations thereof.

20. The method of claims 15-19, including providing the outer coating (16A) of the second electrically conductive material, for the terminal pin (16), having a thickness of from 1.27 X 10⁻⁸ m to 7.62 X 10⁻⁵ m (0.5µ inches to 0.003 inches).

## Patentansprüche

1. Durchführungsanordnung (10), die umfasst:
a) einen Isolator (20) aus elektrisch nicht leitendem Material mit einer Höhe, die durch eine Isolatorseitenwand (24) definiert ist, die sich zu einem ersten Isolatorende (26) und einem zweiten Isolatorende (28) erstreckt, wobei der Isolator wenigstens eine Anschlussstiftbohrung (30) hat, die sich vom ersten Ende bis zum zweiten Ende (28) davon erstreckt;
b) einen Anschlussstift (16), der in der Anschlussstiftbohrung (30) aufgenommen wird, wobei der Anschlussstift (16) eine Seitenwand hat, die sich zu gegenüberliegenden ersten und zweiten Enden erstreckt, die im Abstand von den jeweiligen ersten und zweiten Isolatorenden (26, 28) angeordnet sind; und
c) eine Hülse (18) aus einem elektrisch leitenden Material und die eine Hülsenöffnung umfasst, die durch eine umgebende Seitenwand definiert ist, die sich zu einem ersten Hülsenende und einem zweiten Hülsenende erstreckt, wobei der Isolator (20) in der Hülsenöffnung gelagert ist, **gekennzeichnet durch**
d) den Anschlussstift (16), der einen Anschlussstiftkern (16B) aus einem ersten elektrisch leitenden Material und eine externe äußere Beschichtung (16A) aus einem zweiten elektrisch leitenden Material umfasst, die selektiv auf diskrete Teile des Anschlussstiftkerns (16B) aufgebracht und direkt auf diesen gelagert sind, wobei ein diskreter Teil in einem proximalen Endteil (17) des Anschlussstiftes, das zwischen dem ersten Isolatorende (26) und dem ersten Anschlussstiftende positioniert ist, oder in einem distalen Endteil (21) des Anschlussstiftes liegt, das zwischen dem zweiten Isolatorende (28) und dem zweiten Ende des Anschlussstiftes (16) positioniert ist, wobei die externe äußere Beschichtung (16A) mehrere Zusammensetzungen für jeweils mehrere diskrete Teile umfasst; und
e) ein erstes Lötmaterial, das die Beschichtung des zweiten elektrisch leitenden Materials auf dem Anschlussstiftkern kontaktiert, wobei dadurch der Anschlussstift mit dem Isolator hermetisch dicht verbunden ist, und ein zweites Lötmaterial, das den Isolator mit der Hülse hermetisch dicht verbindet.

2. Durchführungsanordnung (10) nach Anspruch 1, wobei das erste elektrisch leitende Material des Anschlussstiftkerns (16B) aus der Gruppe ausgewählt wird, die aus Niob, Tantal, Nickel-Titan, Titan, insbesondere Beta-Titan, Titanlegierungen, Edelstahl, Molybdän, Wolfram, Platin und Kombinationen davon besteht.

3. Durchführungsanordnung (10) nach Anspruch 1 oder 2, wobei das erste elektrisch leitende Material von der externen äußeren Beschichtung (16A) unbedeckt bleibt, wo das zweite elektrisch leitende Material nicht vorhanden ist.

4. Durchführungsanordnung (10) nach den Ansprüchen 1 - 3, wobei das zweite elektrisch leitende Material 5 Prozent bis 15 Prozent einer Gesamtlänge des Anschlussstiftes (16) kontaktiert, in dem es sich befindet, wobei sich wenigstens ein Teil entweder am proximalen oder distalen Ende des Anschlussstiftes befindet.

5. Durchführungsanordnung (10) nach den Ansprüchen 1 - 3, wobei das zweite elektrisch leitende Material 10 Prozent bis 40 Prozent einer Gesamtlänge des Anschlussstiftes (16) kontaktiert, wo es mit dem Isolator (20) hermetisch dicht verbunden ist.

6. Durchführungsanordnung (10) nach den Ansprüchen 1 - 3, wobei das zweite elektrisch leitende Material, das den proximalen Teil (17) kontaktiert, aus einer anderen Zusammensetzung besteht als das zweite elektrisch leitende Material, das den distalen (21) oder zentralen Teil (19) des Anschlussstiftes kontaktiert.

7. Durchführungsanordnung (10) nach den Ansprüchen 1 - 3 oder 5, wobei der Anschlussstiftkern (16B) einen Durchmesser von 5,08 X 10⁻⁵ m bis 5,08 X 10⁻⁴ m (0,002 Zoll bis 0,020 Zoll) aufweist.

8. Durchführungsanordnung (10) nach den Ansprüchen 1 - 3, 5 oder 7, wobei das zweite elektrisch leitende Material ein Metall ist, das aus der Gruppe ausgewählt wird, die aus Palladium, Palladiumlegierungen, Platin, Gold, Silber, Nickel und Kombinationen davon besteht.

9. Durchführungsanordnung (10) nach den Ansprüchen 1 - 3, 5 oder 7 - 8, wobei die externe äußere Beschichtung (16A) des zweiten elektrisch leitenden Materials des Anschlussstiftes (16) eine Dicke von 1,27 X 10⁻⁸ m bis 7,62 X 10⁻⁵ m (0,5 µZoll bis 0,003 Zoll) aufweist.

10. Durchführungsanordnung (10) nach den Ansprüchen 1 - 3, 5 oder 7 - 9, wobei der Anschlussstift (16) eine Querschnittsform hat, die aus der Gruppe ausgewählt wird, die aus kreisförmig, quadratisch, rechteckig und hexagonal besteht.

11. Durchführungsanordnung (10) nach den Ansprüchen 1 - 3, 5 oder 7 - 10, wobei der Isolator (20) aus der Gruppe ausgewählt wird, die aus Aluminiumoxid, Zirkonoxid, mit Zirkonoxid gehärtetem Aluminiumoxid, Aluminiumnitrid, Bornitrid, Siliziumkarbid, Glas und Mischungen davon besteht.

12. Durchführungsanordnung (10) nach den Ansprüchen 1 - 3, 5 oder 7 - 11, wobei das elektrisch leitende Material der Hülse (18) aus der Gruppe ausgewählt wird, die aus Titan, Tantal, Niob, Edelstahl und Kombinationen von deren Legierungen besteht.

13. Durchführungsanordnung (10) nach den Ansprüchen 1 - 3, 5 oder 7 - 12, wobei das erste und zweite Lötmaterial (34) aus der Gruppe ausgewählt wird, die aus Gold, Goldlegierungen und Silber besteht.

14. Durchführungsanordnung (10) nach den Ansprüchen 1 - 3, 5 oder 7 - 13, die des Weiteren ein Metallisierungsmaterial (32) aufweist, das die Isolatorseitenwand (24) und die Anschlussstiftbohrung (30) bedeckt, wobei das Metallisierungsmaterial (32) aus der Gruppe ausgewählt wird, die aus Titan, Titannitrid, Titankarbid, Iridium, Iridiumoxid, Niob, Tantal, Tantaloxid, Ruthenium, Rutheniumoxid, Zirkonium, Gold, Palladium, Molybdän, Silber, Platin, Kupfer, Kohlenstoff, Kohlennitrid und Gemischen davon besteht.

15. Verfahren zum Bereitstellen einer Durchführungsanordnung (10) nach einem der Ansprüche 1 - 14, die einen Anschlussstift (16) umfasst, wobei das Verfahren die Schritte umfasst:
a) Bereitstellen eines Anschlussstiftkerns (16B) aus einem ersten elektrisch leitenden Material und
b) Beschichten eines diskreten Teils des Anschlussstiftkerns (16B) mit einer externen äußeren Beschichtung (16A) aus einem zweiten elektrisch leitenden Material auf eine derartige Dicke, dass das zweite elektrisch leitende Material nicht vollständig in das erste elektrisch leitende Material des Anschlussstiftkerns (16B) diffundiert wird, wobei ein diskreter Teil in einem proximalen Endteil des Anschlussstiftes (17), der zwischen dem ersten Isolatorende (26) und dem ersten Ende des Anschlussstiftes (16) positioniert ist, oder in einem distalen Endteil des Anschlussstiftes (21) liegt, der zwischen dem zweiten Isolatorende (28) und dem zweiten Ende des Anschlussstiftes (16) positioniert ist.

16. Verfahren nach Anspruch 15, das das Beschichten des zweiten elektrisch leitenden Materials über dem Anschlussstiftkern (16B) unter Verwendung eines Prozesses aufweist, der aus der Gruppe ausgewählt wird, die aus Sputtern, Elektronenstrahlabscheidung, gepulster Laserabscheidung, Galvanotechnik, stromloser Metallabscheidung, chemischer Dampfabscheidung, Vakuumverdampfung, Dickschichtanwendungsverfahren, Aerosolsprühabscheidung und dünner Ummantelung besteht.

17. Verfahren nach den Ansprüchen 15 oder 16, das das Auswählen des ersten elektrisch leitenden Materials des Anschlussstiftkerns (16B) aus der Gruppe aufweist, die aus Niob, Tantal, Nickel-Titan, Titan, insbesondere Beta-Titan, Titanlegierungen, Edelstahl, Molybdän, Wolfram, Platin und Kombinationen davon besteht.

18. Verfahren nach den Ansprüchen 15 - 17, das das Bereitstellen des Anschlussstiftkerns (16B) mit einem Durchmesser von 5,08 X 10⁻⁵ m bis 5,08 X 10⁻⁴ m (0,002 Zoll bis 0,020 Zoll) aufweist.

19. Verfahren nach den Ansprüchen 15 - 18, das das Bereitstellen der äußeren Beschichtung (16A) des zweiten elektrisch leitenden Materials aufweist, das aus der Gruppe ausgewählt wird, die aus Palladium, Palladiumlegierungen, Gold, Silber, Nickel, Platin und Kombinationen davon besteht.

20. Verfahren nach den Ansprüchen 15 - 19, das das Bereitstellen der äußeren Beschichtung (16A) des zweiten elektrisch leitenden Materials für den Anschlussstift (16) mit einer Dicke von 1,27 X 10⁻⁸ m bis 7,62 X 10⁻⁵ m (0,5 µZoll bis 0,003 Zoll) aufweist.

## Revendications

1. Assemblage traversant (10) qui comporte :
a) un isolant (20) en un matériau électriquement non conducteur ayant une hauteur définie par une paroi latérale d'isolant (24) s'étendant jusqu'à une première extrémité d'isolant (26) et une seconde extrémité d'isolant (28), où l'isolant présente au moins un trou (30) pour une broche d'une borne s'étendant de sa première extrémité à sa seconde extrémité (28);
b) une broche de borne (16) reçue dans le trou (30) de broche de borne, la broche de borne (16) ayant une paroi latérale s'étendant à des première et seconde extrémités opposées disposées de manière espacée respectivement par rapport à la première et la seconde extrémité d'isolant (26, 28) ; et
c) une virole (18) en un matériau électriquement conducteur et comportant une ouverture de virole délimitée par une paroi latérale environnante s'étendant jusqu'à une première extrémité de virole et une seconde extrémité de virole, où l'isolant (20) est maintenu dans l'ouverture de virole, **caractérisé par le fait que**
d) la broche de borne (16) comporte une âme de broche de borne (16B) en un premier matériau électriquement conducteur et un revêtement extérieur externe (16A) en un second matériau électriquement conducteur appliqué de manière sélective et directement soutenu par des parties distinctes de l'âme de broche de borne (16B), où une partie distincte se situe dans une partie d'extrémité proximale (17) de la broche de borne, positionnée entre la première extrémité d'isolant (26) et la première extrémité de broche de borne, ou dans une partie d'extrémité distale (21) de la broche de borne, positionnée entre la seconde extrémité d'isolant (28) et la seconde extrémité de la broche de borne (16), où le revêtement extérieur externe (16A) comprend de multiples compositions respectivement pour de multiples parties distinctes ; et
e) un premier matériau de brasage entrant en contact avec le revêtement en second matériau électriquement conducteur sur l'âme de broche de borne, scellant ainsi hermétiquement la broche de borne par rapport à l'isolant et un second matériau de brasage scellant hermétiquement l'isolant par rapport à la virole.

2. Assemblage traversant (10) selon la revendication 1, où le premier matériau électriquement conducteur de l'âme de broche de borne (16B) est sélectionné parmi le groupe comprenant le niobium, le tantale, le nickel-titane, le titane, en particulier le titane de phase bêta, les alliages de titane, l'acier inoxydable, le molybdène, le tungstène, le platine et leurs combinaisons.

3. Assemblage traversant (10) selon la revendication 1 ou 2, où le premier matériau électriquement conducteur est laissé à découvert par le revêtement extérieur externe (16A) là où le second matériau électriquement conducteur n'est pas présent.

4. Assemblage traversant (10) selon les revendications 1-3, où le second matériau électriquement conducteur entre en contact avec 5 pourcents à 15 pourcents d'une longueur totale de la broche de borne (16) où il se trouve, au moins une partie du matériau se trouvant soit à l'extrémité proximale soit à l'extrémité distale de la broche de borne.

5. Assemblage traversant (10) selon les revendications 1-3, où le second matériau électriquement conducteur entre en contact avec 10 pourcents à 40 pourcents d'une longueur totale de la broche de borne (16), là où elle est hermétiquement scellée par rapport à l'isolant (20).

6. Assemblage traversant (10) selon les revendications 1-3, où le second matériau électriquement conducteur entrant en contact avec la partie proximale (17) présente une composition différente de celle du second matériau électriquement conducteur entrant en contact avec la partie distale (21) ou centrale (19) de la broche de borne.

7. Assemblage traversant (10) selon les revendications 1-3 ou 5, où l'âme de broche de borne (16B) présente un diamètre de 5,08 X 10⁻⁵ m à 5,08 x 10⁻⁴ m (0,002 pouces à 0,020 pouces).

8. Assemblage traversant (10) selon les revendications 1-3, 5 ou 7, où le second matériau électriquement conducteur est un métal sélectionné parmi le groupe comprenant le palladium, les alliages de palladium, le platine, l'or, l'argent, le nickel et leurs combinaisons.

9. Assemblage traversant (10) selon les revendications 1-3, 5 ou 7-8, où le revêtement extérieur externe (16A) en second matériau électriquement conducteur de la broche de borne (16) présente une épaisseur de 1,27 x 10⁻⁸ à 7,62 x 10⁻⁵ m (0,5 µ pouces à 0,003 pouces).

10. Assemblage traversant (10) selon les revendications 1-3, 5 ou 7-9, où la broche de borne (16) présente une forme en section transversale sélectionnée parmi le groupe comprenant une forme circulaire, une forme carrée, une forme rectangulaire et une forme hexagonale.

11. Assemblage traversant (10) selon les revendications 1-3, 5 ou 7-10, où l'isolant (20) est sélectionné parmi le groupe comprenant l'alumine, la zircone, la zircone renforcée en oxyde d'aluminium, le nitrure d'aluminium, le nitrure de bore, le carbure de silicium, le verre et leurs mélanges.

12. Assemblage traversant (10) selon les revendications 1-3, 5 ou 7-11, où le matériau électriquement conducteur de la virole (18) est sélectionné parmi le groupe comprenant le titane, le tantale, le niobium, l'acier inoxydable et des combinaisons de leurs alliages.

13. Assemblage traversant (10) selon les revendications 1-3, 5 ou 7-12, où le premier et le second matériau de brasage (34) sont sélectionnés parmi le groupe comprenant l'or, les alliages d'or et l'argent.

14. Assemblage traversant (10) selon les revendications 1-3, 5 ou 7-13, comprenant en outre un matériau de métallisation (32) couvrant la paroi latérale d'isolant (24) et le trou (30) pour la broche de borne, le matériau de métallisation (32) étant sélectionné parmi le groupe comprenant le titane, le nitrure de titane, le carbure de titane, l'iridium, l'oxyde d'iridium, le niobium, le tantale, l'oxyde de tantale, le ruthénium, l'oxyde de ruthénium, le zirconium, l'or, le palladium, le molybdène, l'argent, le platine, le cuivre, le carbone, le nitrure de carbone et leurs mélanges.

15. Procédé pour fournir un assemblage traversant (10) selon l'une quelconque des revendications 1-14 comportant une broche de borne (16), comportant les étapes consistant à :
a) fournir une âme de broche de borne (16B) en un premier matériau électriquement conducteur ; et
b) revêtir une partie distincte de l'âme de broche de borne (16B) avec un revêtement extérieur externe (16A) en un second matériau électriquement conducteur sur une épaisseur telle que le second matériau électriquement conducteur n'est pas complètement diffusé dans le premier matériau électriquement conducteur de l'âme de broche de borne (16B), où une partie distincte se situe dans une partie d'extrémité proximale (17) de la broche de borne, positionnée entre la première extrémité d'isolant (26) et la première extrémité de broche de borne (16), ou dans une partie d'extrémité distale (21) de la broche de borne, positionnée entre la seconde extrémité d'isolant (28) et la seconde extrémité de la broche de borne (16).

16. Procédé selon la revendication 15, comprenant le dépôt du second matériau électriquement conducteur sur l'âme de broche de borne (16B) en utilisant un procédé sélectionné parmi le groupe comprenant la pulvérisation cathodique, le dépôt par faisceau d'électrons, le dépôt par laser pulsé, la galvanoplastie, le placage auto-catalytique, le dépôt chimique en phase vapeur, l'évaporation sous vide, des procédés d'application de film épais, le dépôt par aérosol et le revêtement mince.

17. Procédé selon la revendication 15 ou 16, comprenant la sélection du premier matériau électriquement conducteur de l'âme de broche de borne (16B) parmi le groupe comprenant le niobium, le tantale, le nickel-titane, le titane, en particulier le titane de phase bêta, les alliages de titane, l'acier inoxydable, le molybdène, le tungstène, le platine et leurs combinaisons.

18. Procédé selon les revendications 15-17, comprenant la mise à disposition de l'âme de broche de borne (16B) présentant un diamètre de 5,08 x 10⁻⁵ m à 5,08 x 10⁻⁴ m (0,002 pouces à 0,020 pouces).

19. Procédé selon les revendications 15-18, comprenant la mise à disposition du revêtement extérieur (16A) en second matériau électriquement conducteur sélectionné parmi le groupe comprenant le palladium, les alliages de palladium, l'or, l'argent, le nickel, le platine et leurs combinaisons.

20. Procédé selon les revendications 15-19, comprenant la mise à disposition du revêtement extérieur (16A) en second matériau électriquement conducteur pour la broche de borne (16) présentant une épaisseur de 1,27 x 10⁻⁸ à 7,62 x 10⁻⁵ m (0,5 µ pouces à 0,003 pouces).
